# EUROPEAN PATENT SPECIFICATION

(11) **EP 1 920 232 B1**
(45) Date of publication and mention of the grant of the patent: **14.04.2010**
(21) Application number: 06758508.3
(22) Date of filing: 24.04.2006
(51) Int. Cl.: G01N 13/00, A61F 13/15, A41D 11/00

(54) **SYSTEM FOR DETECTION AND ANALYSIS OF BIOLOGICAL WASTE SPREAD IN AN UNDERGARMENT**
SYSTEM ZUM NACHWEIS UND ZUR ANALYSE DER AUSBREITUNG BIOLOGISCHER ABFALLPRODUKTE IN UNTERWÄSCHE
SYSTEME PERMETTANT DE DETECTER ET D'ANALYSER DES DECHETS BIOLOGIQUES REPANDUS DANS UN SOUS-VETEMENT

(30) Priority: 31.08.2005 US 216859
(43) Date of publication of application: 14.05.2008
(73) Proprietor: KIMBERLY-CLARK WORLDWIDE, INC., Neenah, WI 54956 (US)
(72) Inventor: ALES III, Thomas, M., Neenah, Wisconsin 54956 (US); COHEN, Jason, C., Appleton, Wisconsin 54915 (US); PILECKY, Robert, C., Oshkosh, Wisconsin 54902 (US); SULLIVAN, Shawn, Neenah, Wisconsin 54956 (US)
(74) Representative: Davies, Christopher Robert
(86) International application number: PCT/US2006/015289
(87) International publication number: WO 2007/027219

(56) References cited:
- EP-A- 0 887 055
- US-A1- 2004 118 224
- US-A1- 2004 118 225

## Description

### Background of the Invention

Consumers desire an undergarment that reduces spread of biological waste in an undergarment, especially on a baby after the baby has had a bowel movement (BM) or has urinated. Accordingly, there have been efforts to develop dependable bench methods for performing real-time quantification of BM spread in an undergarment product during simulated mannequin use in order to improve an undergarment product.

Some bench measurement methods have targeted BM spread in static mannequins. For example, the undergarment product is placed on a static mannequin and insulted with a BM simulant. Allowing for dwell time, the undergarment product is removed from the mannequin for inspection. The inspection is typically performed visually or by photographic records, which does not provide a real-time measure of BM spread. Moreover, analyses of visual and photographic data recorded in these measurements are often time intensive and tedious.

To provide real-time measurements of BM spread within the undergarment product during simulated use, there have been only few practical options such as: video imaging within the undergarment; or sensing technology that utilizes a fundamental sensing parameter to provide a position of BM. However, these bench measurement methods suffer from a number of drawbacks.

Video imaging within the undergarment is problematic for several reasons. First, the only practical method of mounting a camera to record the BM movement is within the mannequin. In order to mount within the mannequin, a clear mannequin material is required, but a clear mannequin material or skin is difficult to work with due to conformability and surface problems. Second, even if a camera is mounted within the clear skin, there are drawbacks associated with flat field imaging. Specifically, a mannequin body exhibits a curvature, which is imposed upon the donned undergarment. Therefore, a single camera cannot reliably image the BM spread without severe distortion to the resulting BM spread images. Moreover, if analysis of BM spread in a moving mannequin is desired, to produce the mannequin to test motion, a skeleton has to be employed to support the mannequin skin. Such a skeleton will interfere with imaging and limit space for camera placement.

Identifying a sensing technology for real-time BM spread requires analysis of features of a system that can be sensed. A component lending itself to detection is the BM simulant. The BM simulant can be sensed by fluid conductivity or interruption of a sensed parameter. Both of these techniques are inherently discrete, which requires several sensors to simultaneously respond over a finite area to detect position of the BM simulant. For fluid conductivity sensing, the BM simulant would have to physically flow between two conducting materials. The conductive fluid would complete a circuit and single that the BM simulant is present in a particular position. Conductivity measurement, however, is problematic in a skin surface sensor array for a variety of reasons. First, there are no commercially available conductivity arrays in a film format with sufficient spatial resolution. The other method of conductivity measurement would be to use a simple conductivity probe constructed of two conductive materials acting as a single point sensor and building an array of probes to look at distribution of the BM simulant. Previous attempts to use this method for urine detection and distribution have been cumbersome and problematic due the size of the conductive surfaces and proximity of the conductive materials. Moreover, loss of spatial resolution and false signaling plagued setups of this type. Additionally, this sensing technique requires that the BM simulant be in contact with the conductivity probe. This may leave BM that spreads in an undergarment undetected if it were not to come in contact with the skin of the mannequin.

A system is needed in the industry for real-time detection and spread analysis of biological waste in an undergarment.

US 2004/0118224 A1 discloses a prior art system and method for evaluating materials for use in absorbent articles. The method comprises placing the material proximate a simulated skin substrate, insulting the material with a fluid and, after a predetermined time, taking fluid loss measurements. US 2004/0118224 A1 discloses the features of the preambles of claims 1 and 13.

EP 0 887 055 A1 discloses a prior art method of monitoring the humidity within an absorbent article after an insult.

### Brief Summary of the Invention

In general, the present invention provides a detection and analysis system using a mannequin motion system equipped with a sensor that senses a parameter. The parameter is interrupted by a biological waste spread to detect and analyzed the spread in real-time. The component parts of the detection and analysis system are simple and economical to manufacture, assemble and use. Other advantages of the invention will be apparent from the following description and the attached drawings, or can be learned through practice of the invention.

As used herein, the term biological waste includes a simulated excrement, a simulated excretory product, a simulated exudate, a simulated bowel movement (BM), simulated feces, simulated urine, simulated menses, simulated blood and any other simulated human waste excretion.

As used herein, the terms simulated and simulant are used to refer to any manufactured materials, objects and fluids and can mean virtual, artificial, synthetic and the like.

As used herein, the term undergarment means any diaper, childcare, feminine care, adult care, healthcare or other undergarment.

According to one aspect of the invention, a system for analyzing biological waste in an undergarment is provided, as set forth in claim 1.

In one embodiment, the mannequin simulates human motion in order to better simulate biological waste spread such as when a toddler walks around a room. The evacuation port of the mannequin can simulate a human anus with the excretory product being a feces simulant for elimination through the simulated anus. Also in this aspect, an insult port can be provided for insulting simulated human urine from the mannequin into the undergarment is provided.

At least one of the sensors in this aspect of the invention is a photodiode disposed in the skin surface of the mannequin. A light source can be located about the mannequin to produce the light emission to activate the photodiode. The photodiode senses a light emission external to the skin surface and signals the computer when the excretory product is affecting the light emission. More particularly, the photodiode can be substantially flush with the skin surface, and is disposed under a transparent polymer coating, which itself is coated on the skin surface. The excretory product can be opaque in appearance to block the light emission, but it can also be translucent to alter the light emission. Likewise, the simulated human urine can include a darkening agent to affect sensing by the sensors.

At least one of the sensors in this aspect of the invention can be a heat sensor disposed in the mannequin at least partially exposed to an ambient environment. The heat sensor senses a presence of the excretory product when a temperature of the excretory product is greater than an ambient temperature in the ambient environment.

At least one of the sensors can also be a humidity sensor disposed in the mannequin. The humidity sensor senses a humidity of the excretory product.

The computer records the dispersion pattern being produced by the excretory product being sensed by the sensors. The computer includes means for replaying the recorded dispersion pattern for a user to analyze the dispersion pattern being formed in real-time. Additionally, the system can include means for programming the computer to convert the excretory product into the dispersion pattern. The means for programming can be a software program configured to record the dispersion pattern in real-time and replay the dispersion pattern for analysis.

According to another aspect of the invention, a method is provided, as set forth in claim 13.

The method can also include the step of injecting a darkening agent in the excretory product such that the excretory product exhibits an opaque appearance to block the emission.

The method can also accommodate the step of placing the mannequin in motion while the excretory product is being eliminated from the evacuation port into the undergarment.

Other aspects and advantages of the invention will be apparent from the following description and the attached drawings, or can be learned through practice of the invention.

### Brief Description of the Drawings

A full and enabling disclosure of the present invention, including the best mode thereof to one of ordinary skill in the art, is set forth more particularly in the remainder of the specification, including reference to the accompanying figures in which:
FIGURE 1 is a perspective view of a detection and analysis system according to one embodiment of the invention;
FIGURE 2 is a schematic view of a portion of the detection and analysis system as in FIGURE 1;
FIGURE 3A is a top perspective view of a mannequin as used in the detection and analysis system of FIGURE 2;
FIGURE 3B is a bottom perspective view of the mannequin as in FIGURE 3A;
FIGURE 4 is an elevational view of a photodiode as used in one embodiment of the invention;
FIGURE 5 is a schematic diagram showing operation of the photodiode as in FIGURE 4;
FIGURE 6 is a schematic diagram showing placement points in the mannequin for the photodiodes of FIGURE 4;
FIGURE 7 is an elevational view of a display showing an intensity graph relative to the schematic diagram of FIGURE 6;
FIGURE 8 is a flow chart of a computer program for recording real-time biological waste spread in accordance with an aspect of the invention; and
FIGURE 9 is a flow chart of a computer program for playing back a recorded biological waste spread as in FIGURE 8.

### Detailed Description of the Invention

Detailed reference will now be made to the drawings in which examples embodying the present invention are shown. Repeat use of reference characters in the drawings and detailed description is intended to represent like or analogous features or elements of the present invention.

The drawings and detailed description provide a full and detailed written description of the invention and the manner and process of making and using it, so as to enable one skilled in the pertinent art to make and use it. The drawings and detailed description also provide the best mode of carrying out the invention. However, the examples set forth herein are provided by way of explanation of the invention and are not meant as limitations of the invention. The present invention thus includes modifications and variations of the following examples as come within the scope of the appended claims and their equivalents.

As broadly embodied in the figures, a detection and analysis system is provided with sensors that sense a parameter, which can be interrupted by a simulated biological waste in an undergarment for detection and analysis of a spread of the simulated biological waste. The detection and analysis system broadly includes a mannequin motion system with a movable mannequin connected to a movement controller within a light box. The mannequin is in electronic communication with a computer for relaying collected data relative to the mannequin. The skilled artisan will instantly recognize that the components of the detection and analysis system, described in detail below and their materials and dimensions are modifiable to accommodate various manufacturing and testing requirements and are limited to only those examples shown in the figures.

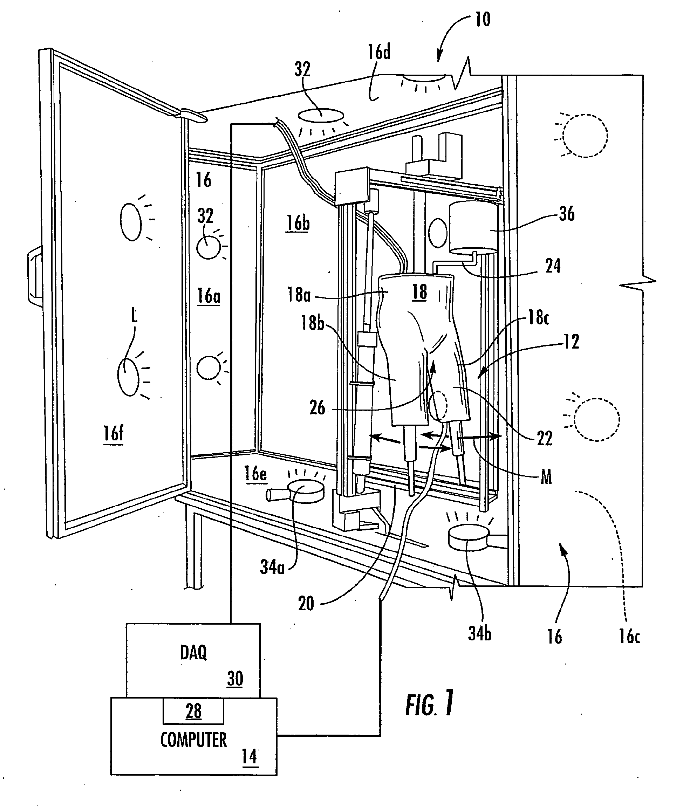

A first embodiment of a detection and analysis system, designated in general by the element number 10, is shown in FIGS. 1-5. The detection and analysis system 10 broadly includes a mannequin motion system 12, a computer 14, a light box 16, a mannequin 18 and a controller 20. By way of brief introduction, the mannequin 18 is electrically connected to the computer 14 and the controller 20 within the light box 16. An undergarment U (shown in phantom for clarity) is attached to the mannequin 18 in a known manner and a simulated biological waste product B is insulted through the mannequin 18 into the undergarment U. As will be described in greater detail below, a number of sensors such as photodiodes 40 are embedded in or located near a surface or simulated skin 22 of the mannequin 18. As the biological waste B passes over the photodiodes 40, the photodiodes 40 register the presence of the biological waste B due to an attenuation of incoming light that is normally received by the photodiodes 40. Further details of the photodiodes and alternative sensors as well as other components of the detection and analysis system 10, its material makeup, arrangement and examples of its operation are provided in greater detail below.

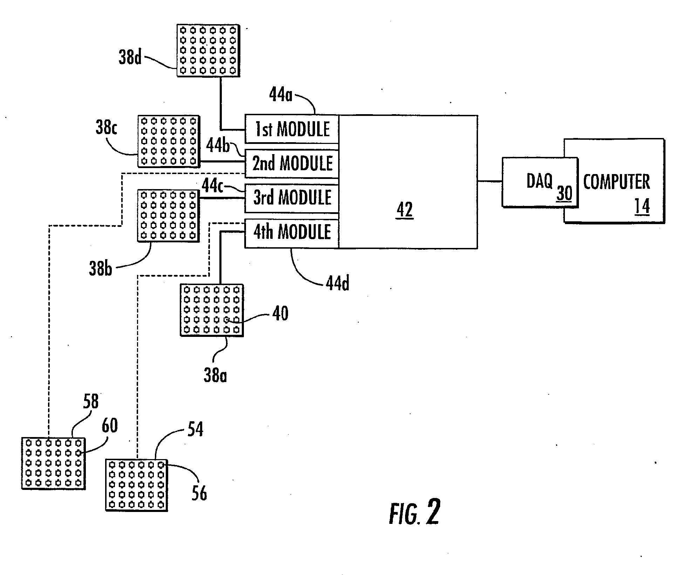

With particular reference to FIGURES 1 and 2, the detection and analysis system 10 utilizes the light box 16 with a plurality of sensors such as photodiodes 40a-x (where x represents a theoretically limitless number of photodiodes), which are situated in the mannequin 18. As discussed in greater detail below, in addition to the photodiodes 40a-x, or alternatively, a plurality of heat sensors 56a-x and humidity sensors 60a-x can be used by the detection and analysis system 10.

As shown most clearly in FIGURE 1, the mannequin 18 is positioned within the light box 16, which includes three walls 16A-C, a ceiling 16D, a floor 16E and a door 16F. As shown, the mannequin 18 includes a simulated skin 22 and forms a torso 18A and left and right legs 18B,C. The mannequin 18 also includes an evacuation port 26 (alternatively, insult port or aperture) defined through the simulated skin 22. The legs 18A,B are connected to the controller 20 to control movement of the mannequin 18 as will be described below. As further shown, a biological waste simulant container 36 is connected to the mannequin 18 via a tube 24 that extends to the evacuation port 26. The mannequin 18 is also connected to the computer 14 and a data acquisition card (DAQ) 30. The DAQ 30 collects data on the simulated biological waste B spread in the undergarment U and displays that data on a display 28 as will be described below.

FIGURE 1 further shows a plurality of mounted light fixtures 32a-x (where x represents a theoretically unlimited number of fixtures). Also shown, at least two mobile fixtures 34a,b known as "trouble lights" can be positioned as desired to change an emission of light rays L or intensity relative to certain aspects of the mannequin 18. FIGURE 1 also illustrates potential directions of motion by the legs 18A,B via the controller 20 as indicated by the bold arrows labeled M.

Turning now to FIGURE 2, a portion of the detection and analysis system 10 is shown schematically. As shown, the computer 14 is interfaced to a multiplexer 42 such as a four slot SCXI series chassis available from National Instruments Corporation, Austin, Texas. As shown, four (4) photodiode arrays 38a-d, each having thirty-two photodiodes 40a-x, are interfaced to respective modules 44a-d, which are connected to the multiplexer 42. In this aspect of the invention, the DAQ 30 is an NI PCI-6036e card also available from National Instruments Corporation. The National Instruments DAQ and related system is suitable due to flexibility, expandability, accuracy and programming ease. For instance, the NI PCI-6036e card provides sixteen-bit accuracy (greater than 65,000 levels) on analog inputs at 200-kilo samples/second. However, since the NI PCI-6036e card allows only sixteen single-ended or eight differential inputs, a signal conditioning/multiplexing chassis, the multiplexer 42, is used to expand the range of the DAQ 30 in this exemplary set up. The skilled artisan will instantly recognize that other data acquisition systems and cards can be used; therefore, the DAQ 30 of the detection and analysis system 10 is not limited to the NI PCI-6036e card, which is an example only. Moreover, use of a greater capacity acquisition card that allows more than sixteen single-ended or eight differential inputs can eliminate the need for the multiplexer 42 as used in this example.

With continued reference to FIGURE 2, the multiplexer 42 is shown loaded with the four modules 44A-D. The modules 44A-D are each SCXI-1032 channel analog input modules that provide a total of 128 analog input channels. The 128 channels are multiplexed by the multiplexer 42 to the DAQ card 30 channels through the multiplexer 42. The modules 44A-D are connectible to the photodiode arrays 38A-D through removable shielded terminal blocks (not shown).

FIGURE 2 further shows the plurality of heat sensors 56a-x and humidity sensors 60a-x briefly introduced above. As shown, a heat sensor array 54 includes the plurality of heat sensors 56a-x. The heat sensors 56a-x are interfaced to one of the modules 44a-d, which are connected to the multiplexer 42 as described above. Likewise, a humidity sensor array 58 includes the plurality of humidity sensors 60a-x, which is also connected to the multiplexer 42. For clarity, only one heat sensor array 54 and only one humidity sensor array 58 are shown in FIGURE 2, but those skilled in the art will recognize that multiple arrays, each containing multiple sensors, can be employed in the detection and analysis system 10. The heat sensors 56a-x and the humidity sensors 60a-x can be physically arranged on, in or about the mannequin 18 for sensing heat and humidity, respectively, in real time in a manner similar to the photodiodes 40a-x as discussed below.

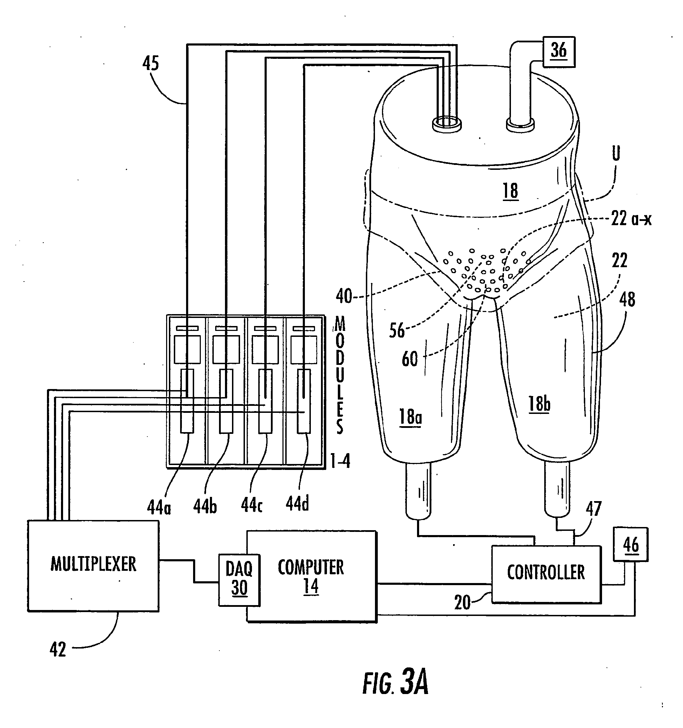

Turning now to FIGURE 3A, the mannequin 18 is more particularly shown connected to the controller 20 and the multiplexer 42, which are powered by a power supply 46. As shown, a plurality of recesses 22a-x are defined in the simulated skin 22 of the mannequin 18. In this example, the simulated skin 22 is a polymer although various elastomeric materials can be used, which have sufficient extensibility and elastic recovery properties. A plurality of electrical connecting wires 45 is used to connect the sensors such as the photodiodes 40a-x to the modules 44a-x, which are connected to the multiplexer 42. The wires 45 can be two-conductor, sealed wire obtained from manufacturers such as Bay Associates of Menlo Park, California, which can custom create the wires 45 to be small, flexible 36 AWG gauge wire. As further shown, the legs 18B,C are connected by cables/serial ports 47 to the computer 14 as known to the skilled artisan, and the electrical connecting wires 45 connect the controller 20 to the power supply 46 to control movement of the mannequin 18.

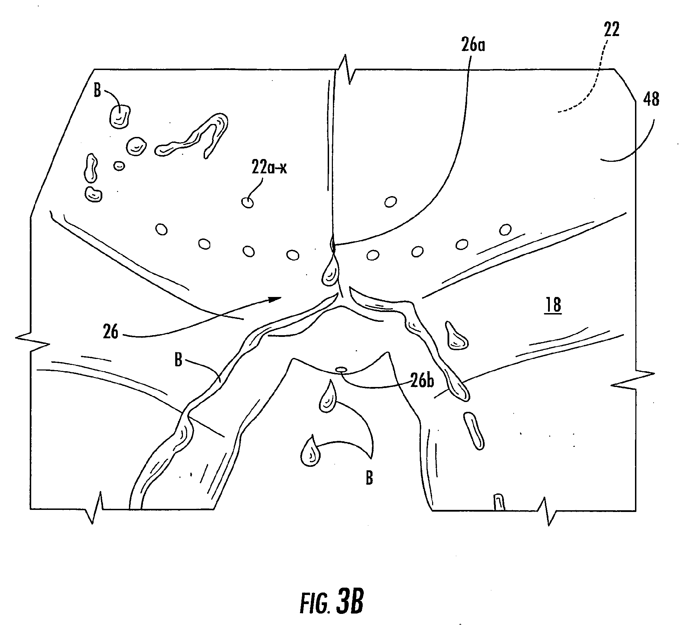

With reference to both FIGURES 3A and 3B, the recesses 22a-x are used to position the photodiodes 40a-x, the heat sensors 56a-x, and the humidity sensors 60a-x, as well as other sensors and combinations of these sensors, in or on the mannequin 18. As FIGURE 3B particularly shows, the evacuation port 26 can be a simulated anus 26A or a simulated urethra 26B. To prevent the simulated biological waste B from flowing into the recesses 22a-x from the evacuation port 26 and contacting the sensors or the electrical connecting wires 45, a protective compound or covering 48 is used to coat the mannequin 18. The covering 48 can be a silicone rubber compound such as Dragon Skin™ brand available from Smooth On, Inc. Such a silicone rubber compound is also durable and flexible, which is useful for repetitive motions when the mannequin 18 is put into motion by the controller 20.

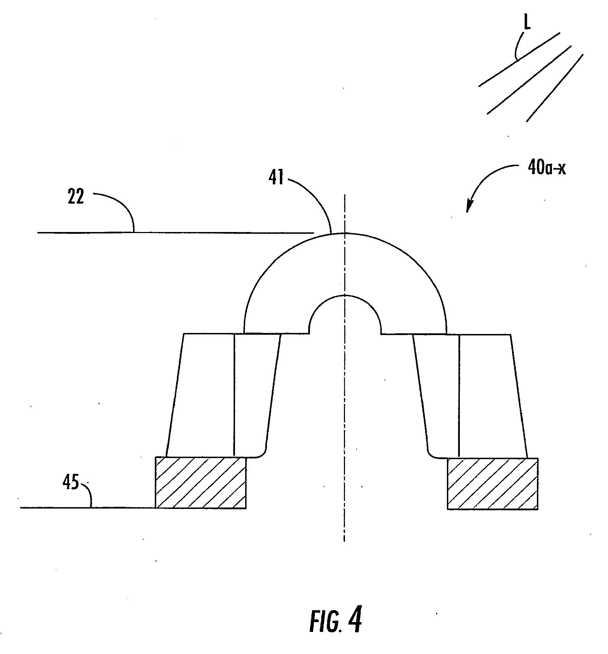

Turning now to FIGURE 4, one sensor according to an aspect of the invention is the photodiode 40a-x as briefly introduced above. The photodiode 40a-x includes an N and P type silicone sandwiched together for exposure to ambient lighting such as the mounted fixtures 32a-x and mobile fixtures 34A,B as described above. The photodiodes 40 each have a lens 41 for passage of the light rays L. In this example, the lens 41 is located near the skin surface 22 as described above. In use, when a reverse bias voltage is applied to the photodiode 40, a "depletion region" (lack of electrical charge) is created in the PN junction. As photons of certain wavelengths from the light rays L fall on the depletion region, an electron hole pair is created. The electron hole pair separate with an electron entering the N-type silicone and the hole entering the P-type, which results in a current generated by the light rays incident upon the photodiode 40. Migration of holes and electrons to their respective regions is known as the photovoltaic effect.

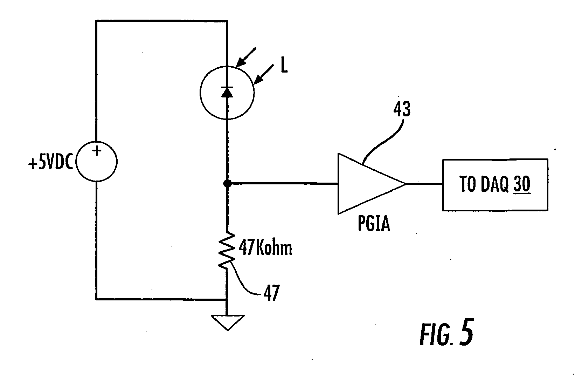

With reference to FIGURES 4 and 5, a basic circuit to read a current from the photodiode 40a-x is shown. As introduced above, the light rays L reach the lens 41 of the photodiode 40a-x to allow current to flow that is proportional to intensity and wavelength of the light rays L. In this aspect of the invention, the input to a programmable gain instrumentation amp (PGIA) 43 located on the analog input module has relatively high impedance; thus, most of the current flows through a Kilo-ohm resistor 47 as shown, which creates a voltage drop. This voltage is amplified and multiplexed back to the DAQ card 30 where the voltage drop is then reported to software loaded in the computer 14, which will be described below.

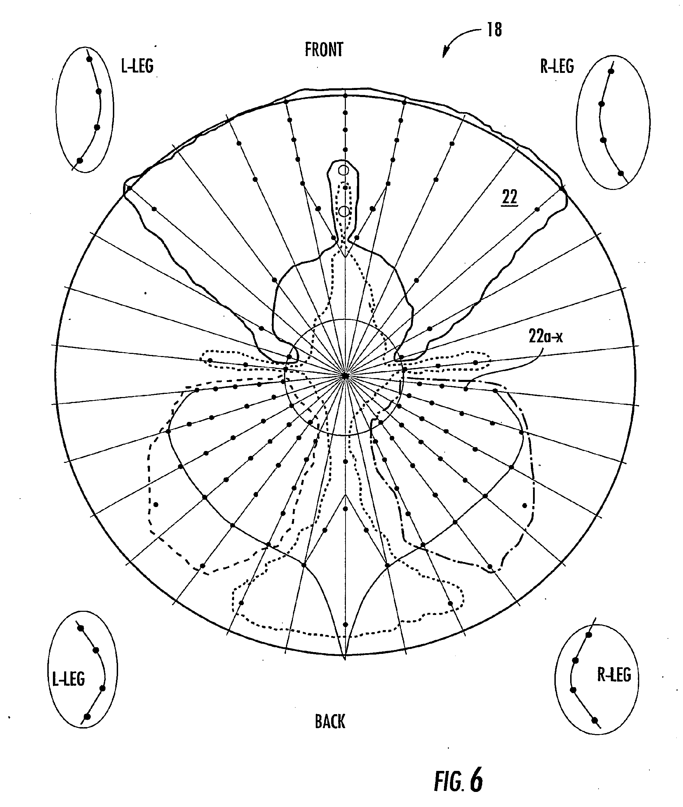

FIGURE 6 most clearly shows the plurality of photodiode recesses 22a-x in the skin 22 of the mannequin 18 arranged for detecting and monitoring the simulated biological waste B spread when the undergarment U is placed around the mannequin 18 in a typical fashion. As noted above, the lens 41 of each photodiode 40a-x rests in, or possibly slightly protrudes from, the photodiode recesses 22a-x. More particularly, each lens 41 is substantially flush with the skin 22 as will be described example operation below.

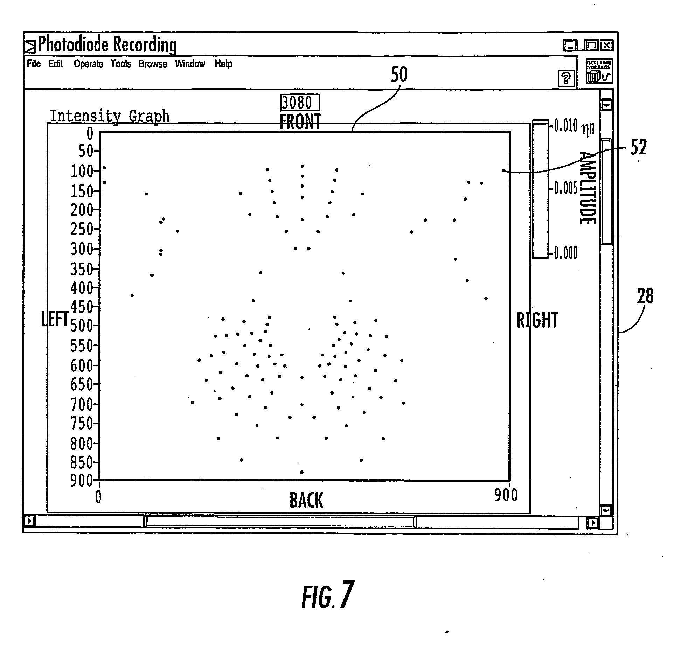

FIGURE 7 most clearly shows the display 28 of the computer 14 as briefly introduced above. As shown, a two-dimensional intensity graph 50 related to a sensed output of the photodiodes 40a-x (see FIGURE 4) is shown. Individual photodiode intensity markers 52 are on the order of a picture element (pixel).

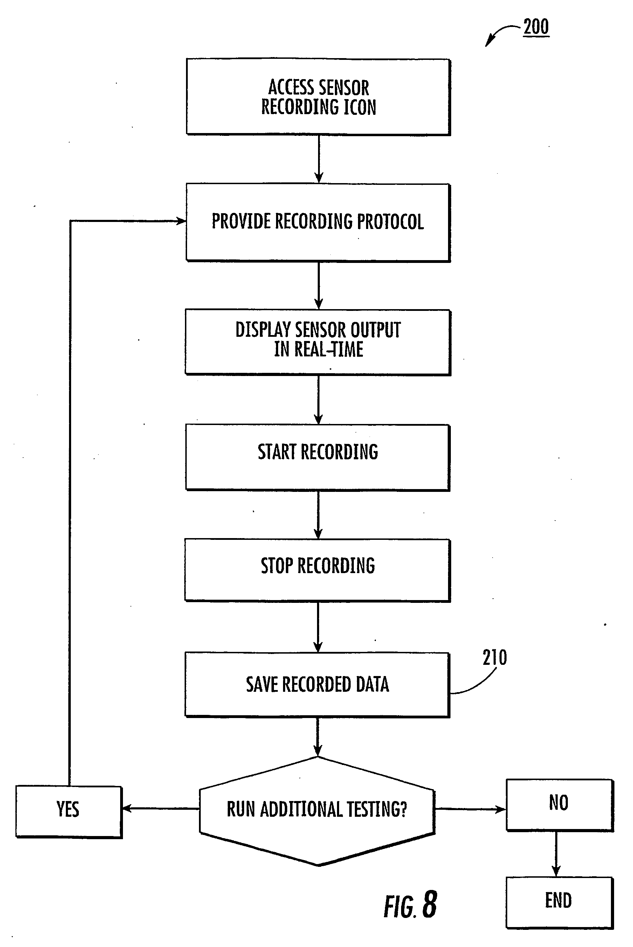

FIGURE 8 shows creation of a data-recording program for use with the detection and analysis system 10. In this aspect of the invention, LabView® programming language, available from National Instruments Corporation, is used to create the recording program. The recording program is used to record incoming data from sensors such as the photodiodes 40a-x as described above with respect to FIGURES 1-7. An output of the multiplexer 42 is recorded using, by example, a LabView® data acquisition subroutine. The subroutine allows for the analog-to-digital conversion-of the sensor input so that the output can be recorded and displayed within the LabView® program (see FIGURE 7).

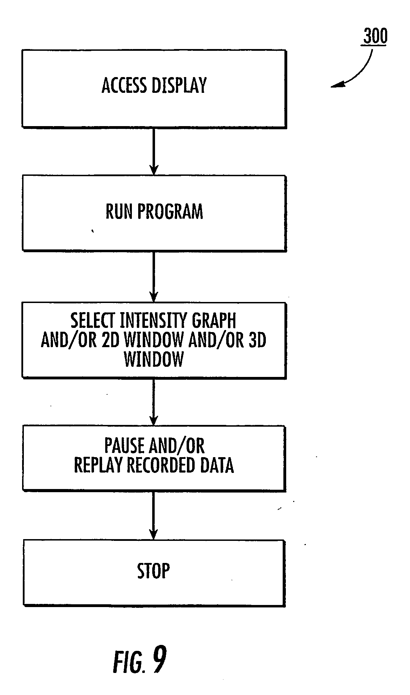

Turning now to FIGURE 9, a second program is required to play back previously recorded information as described above with respect to FIGURE 8. The saved data from the sensors such as the photodiodes 40a-x can be displayed in a 900 x 900 intensity graph and/or as shown in FIGURE 7 to watch the spread of the simulated biological waste B as measured by changes in humidity, temperature or the photodiode intensity. The playback program according to FIGURE 9 can also include the intensity graph to show an X-Y streaming output that changes in amplitude and color intensity as the sensor voltage changes.

The invention may be better understood with reference to an exemplary operation of the detection and analysis system 10 as described above.

With particular reference to FIGURES 1-5, the mannequin 18 is connected to the controller 20 and to the computer 14 from within the light box 16.

### EXAMPLE

For first-use evaluation, the mannequin 18 was left in a static position and signal levels of the photodiodes 40a-x were observed in two situations; i.e., with and without a product U donned. The signal level of the photodiodes 40a-x without the product U was approximately 1 V. With the product U donned, the signal levels were approximately an order of magnitude less. This was dependant on the location of the photodiode 40a-x with respect to the product U. The photodiodes 40a-x that are at the front waist region of the mannequin are severely attenuated due to the opaque nature of the PUB.material landing zone of the product U. Also in the crotch region of the product U, there is extensive bunching and overlapping of cover/core materials, which attenuates the light entering. Higher wattage light sources 32a-x can be used to increase the depth of penetration of light into the product U. The caveat to the higher wattage sources 32a-x is the higher heat conditions inside of the box 16. The distance of the sources 32a-x from the photodiodes 40a-x will also play a part in the flux of light though the product U. The inverse square law for radiant sources must be considered for this setup. The law states that the intensity from the radiant source will diminish with relation to the inverse square of the distance. Therefore, the farther removed the lighting 32a-x, the larger the attenuation of intensity. Customized lighting, such as trouble lights 34a,b is flexible and invasive enough to eliminate this attenuation problem.

The mannequin 18 was run through some simple non-repetitive dynamic motions to see the effects on the photodiodes 40a-x. Once the Dragon Skin^{®} was put in place on the mannequin 18, there was not immediate movement of the photodiodes 40a-x from the surface of the mannequin 18. Due to the rigidity of the heat shrink on the outside of the photodiode connection wires 45, it was suspected that the dynamics of the mannequin 18 may cause the photodiodes 40a-x and the wires 45 to come out of the surface of the mannequin 18. This may have been remedied due to two reasons. First, despite the mold release that was placed on the heat shrink of the wires 45, the wires 45 were probably molded in curved positions and this would prevent an angular placement of the wire 45 (with respects to the normal of the skin surface 22) to allow for it to be mobile within the mannequin 18. Second, the clear skin 22 may act as a barrier that holds the photodiodes 40a-x in place. When placed on the mannequin 18, the skin 22 filled in around the photodiodes 40a-x and into the holes 22a-x of the mannequin 18. This may also help the stability of the photodiodes 40a-x in the mannequin 18.

When donned with the product U and insulted with 60 mL of 10-3 simulant, the photodiode mannequin 18 clearly indicated the presence of a simulated bowel movement (BM) or simulant B when covered. An additional observation that was made during the testing was that at the edge of the insult spread region, the output signal of the photodiode 40a-x did not drop suddenly. Photodiodes 40a-x at the edge of the BM slowly decreased in output until covered by enough simulant B to block all extent light. This indicates the photodiodes 40a-x are sensitive to thickness changes in the BM to a point. Alternatively stated, in addition to the photodiodes 40a-x being able to detect varying light intensities, the photodiodes 40a-x are also capable of assessing thickness of the simulant B covering one or more of the photodiodes 40a-x when the simulant B is thin enough and/or translucent to allow some light to pass.

To prevent moisture from contacting the edges of the clear skin 22 at the point of interface with the mannequin surface 18, the edges of clear skin 22 were painted to the top 18A of the torso 18 and down to middle of the legs 18B,C. Around the BM insult point 26, the clear skin 22 was placed into the hole surrounding the insult tube 26 and onto the tube. A sized o-ring was then slipped over the tube 26 to hold the skin 22 in place in case peeling occurred. The Dragon Skin^{®} works as a natural buffer for both the mannequin 18 and photodiodes 40a-x. If the skin 22 were to become stained with BM to a point where cleaning becomes problematic or begins to peel in the insult region, the skin can be easily stripped from the mannequin 18 and replaced.

The original design of the light box 16 incorporated fluorescent lights due to their higher lumen output and temperature regulation. Despite spectral sensitivity data indicating otherwise, the photodiodes 40a-x did not provide an adequate output when exposed to fluorescent lighting. To correct this, the box 16 was retrofitted with incandescent bulbs 32a-x since the spectral sensitivity of the photodiodes 40a-x peaks in the high visible to near infrared region of the electromagnetic spectrum. This retrofit provided sufficient excitation for the photodiodes 40a-x. The temperature due to the incandescent bulbs 32a-x varied in the range of 83 to 84 degrees F after one hour of warm up and one hour of monitoring. Therefore, the temperature of the box 16 is acceptable and will create more real conditions (biological temperatures) for testing.

While the exemplary software used in the foregoing testing was capable of recording and subsequently playing back the recorded data, real-time playback speed can be increased in order for a user to more quickly observe the data as it is being collected. Therefore, other software can be substituted to permit even more rapid displays of large intensity graphs in order to improve the real-time display ability of the system 10. Additionally, alternative software can be loaded in the computer 14 to provide three-dimensional analyses of biological waste spread. Such "3D" software is available, for instance, from National Instruments Corporation to enable a technician to operate the computer 14 in a manner similar to that described above with respect to FIGS. 7-9. Specifically, by selecting the 3D window in FIG. 9, the technician can rotate the individual photodiode intensity markers 52 about X-Y-Z axes on the display 28 of the computer 14 to present different aspects of the mannequin 18 in order to analyze the spread at particular points on the mannequin 18.

## Claims

1. A system (10) for analyzing biological waste in an undergarment, the system (10) comprising:
a mannequin (18) having a simulated skin surface (22) and defining an evacuation port (26) therethrough, the evacuation port (26) being configured to eliminate a simulated human excretory product from the mannequin (18); **characterised by**
a plurality of sensors disposed about the evacuation port (26), each sensor being configured for sensing the excretory product as the excretory product is eliminated through the evacuation port (26) into an undergarment disposed about the mannequin (18); and
a computer (14) in communication with the sensors, the computer (14) being configured for converting the sensed excretory product into a dispersion pattern defined in the undergarment;
wherein at least one of the sensors is a photodiode (40a-x) disposed in the skin surface (22) of the mannequin (18), the photodiode (40a-x) being configured to sense a light emission external to the skin surface (22), the photodiode (40a-x) being further configured to signal the computer (14) when the excretory product is affecting the light emission, and wherein the photodiode (40a-x) is disposed under a transparent polymer coating, the transparent polymer coating disposed on the skin surface (22).

2. The system (10) as defined in Claim 1, wherein the mannequin (18) is configured to simulate human motion.

3. The system (10) as defined in Claim 1 or 2, wherein the photodiode (40a-x) is substantially flush with the skin surface (22).

4. The system (10) as defined in any one of the preceding claims, wherein the evacuation port (26) is selected from the group consisting of a simulated anus, a simulated urethra, and combinations thereof.

5. The system (10) as defined in Claims 3 or 4, wherein the excretory product is opaque in appearance to block the light emission, or translucent in appearance to alter the light emission.

6. The system (10) as defined in any one of the preceding claims, wherein the excretory product is one of a feces simulant or a menses simulant

7. The system (10) as defined in any one of the Claims 3-6, further comprising a light source disposed about the mannequin (18), the light source being configured to produce the light emission to activate the photodiode (40a-x).

8. The system (10) as defined in any one of the preceding claims, wherein at least one of the sensors is a heat sensor (56a-x) disposed in the mannequin (18) at least partially exposed to an ambient environment, the heat sensor (56a-x) being configured to sense a presence of the excretory product when a temperature of the excretory product is greater than an ambient temperature in the ambient environment, or at least one of the sensors is a humidity sensor (60a-x) disposed in the mannequin (18), the humidity sensor (60a-x) being configured to sense a humidity of the excretory product.

9. The system (10) as defined in any one of the preceding claims, wherein the computer (14) is configured to record the dispersion pattern being produced by the excretory product being sensed by the sensors.

10. The system (10) as defined in Claim 9, wherein the computer (14) includes means for replaying the recorded dispersion pattern for a user to analyze the dispersion pattern being formed in real-time.

11. The system (10) as defined in Claims 1, 9 or 10, further comprising means for programming the computer (14) to convert the excretory product into the dispersion pattern, preferably the means for programming includes a software program being configured to record the dispersion pattern in real-time and replay the dispersion pattern for analysis.

12. The system (10) as defined in any one of the preceding claims, further comprising an insult port being configured to insult simulated human urine from the mannequin (18) into the undergarment, preferably the simulated human urine includes a darkening agent to affect sensing by the sensors.

13. A method of determining biological waste dispersion in an undergarment, the method comprising the steps of:
providing a mannequin (18) defining an evacuation port (26) therethrough, the evacuation port (26) being configured to eliminate a simulated human excretory product from the mannequin (18);
**characterised by** the steps of:
disposing a plurality of sensors about the evacuation port (26), each sensor being configured for sensing the excretory product as the excretory product is eliminated from the evacuation port (26) into an undergarment disposed about the mannequin (18), wherein at least one of the sensors is a photodiode (40a-x) substantially flush with a simulated skin surface (22) of the mannequin (18) such that an emission from a light source is affected by the excretory product as the excretory product moves between the photodiode (40a-x) and the light source, and wherein the photodiode (40a-x) is disposed under a transparent coating, the transparent coating disposed about the simulated skin surface (22); and
providing a computer (14) in communication with the sensors, the computer (14) being configured for converting the sensed excretory product into a dispersion pattern defined in the undergarment.

14. The method as defined in Claim 13, wherein the sensors are a plurality of photodiodes (40a-x), a plurality of heat sensors (56a-x), a plurality of humidity sensors (60a-x) or a combination thereof.

15. The method as defined in Claims 13 or 14, further comprising the step of injecting a darkening agent in the excretory product such that the excretory product exhibits an opaque appearance to block the emission.

16. The method as defined in any one of the Claims 13-15, further comprising the steps of recording a dispersion pattern of the excretory product being sensed by the sensors and replaying the dispersion pattern of the excretory product for a user to analyze the dispersion pattern.

17. The method as defined in any one of the Claims 13-16, further comprising the step of placing the mannequin (18) in motion while the excretory product is being eliminated from the evacuation port (26) into the undergarment.

## Patentansprüche

1. System (10) zum Analysieren von biologischen Abfallprodukten in einer Unterwäsche, wobei das System (10) umfasst:
eine Puppe (18), welche eine simulierte Hautoberfläche (22) aufweist und dahindurch eine Entleerungsöffnung (26) definiert, wobei die Entleerungsöffnung (26) gestaltet ist, um ein simuliertes menschliches Exkretionsprodukt aus der Puppe (18) auszuscheiden; **gekennzeichnet durch**
eine Vielzahl an Sensoren, die um die Entleerungsöffnung (26) angeordnet sind, wobei jeder Sensor eingerichtet ist, das Exkretionsprodukt wahrzunehmen, wenn das Exkretionsprodukt **durch** die Entleerungsöffnung (26) in eine Unterwäsche ausgeschieden wird, welche um die Puppe (18) angeordnet ist; und
einen Computer (14) in Kommunikation mit den Sensoren, wobei der Computer (14) eingerichtet ist, das wahrgenommene Exkretionsprodukt in ein Verteilungsmuster umzuwandeln, welches in der Unterwäsche definiert ist;
wobei mindestens einer der Sensoren eine Photodiode (40a-x) ist, welche in der Hautoberfläche (22) der Puppe (18) angeordnet ist, wobei die Photodiode (40a-x) eingerichtet ist, eine Lichtemission außerhalb der Hautoberfläche (22) wahrzunehmen, wobei die Photodiode (40a-x) des Weiteren eingerichtet ist, dem Computer (14) zu signalisieren, wenn das Exkretionsprodukt die Lichtemission beeinflusst, und wobei die Photodiode (40a-x) unter einer transparenten Polymerbeschichtung angeordnet ist, wobei die transparente Polymerbeschichtung auf der Hautoberfläche (22) angeordnet ist.

2. Das System (10) gemäß Anspruch 1, wobei die Puppe (18) eingerichtet ist, menschliche Bewegung zu simulieren.

3. Das System (10) gemäß Anspruch 1 oder 2, wobei die Photodiode (40a-x) im Wesentlichen bündig mit der Hautoberfläche (22) ist.

4. Das System (10) gemäß einem der vorherigen Ansprüche, wobei die Entleerungsöffnung (26) ausgewählt ist aus der Gruppe bestehend aus einem simuliertem Anus, einer simulierten Urethra und Kombinationen davon.

5. Das System (10) gemäß Anspruch 3 oder 4, wobei das Exkretionsprodukt eine opake Erscheinung aufweist, um die Lichtemission zu blocken, oder eine transluzent Erscheinung aufweist, um die Lichtemission zu verändern.

6. Das System (10) gemäß einem der vorherigen Ansprüche, wobei das Exkretionsprodukt eines ist aus simulierten Fäzes oder simulierten Menses.

7. Das System (10) gemäß einem der Ansprüche 3-6, welches des Weiteren eine Lichtquelle umfasst, die um die Puppe (18) angeordnet ist, wobei die Lichtquelle eingerichtet ist, Lichtemission zu erzeugen, um die Photodiode (40a-x) zu aktivieren.

8. Das System (10) gemäß einem der vorherigen Ansprüche, wobei mindestens einer der Sensoren ein Wärmesensor (56a-x) ist, der in der Puppe (18) zumindest zum Teil einer Raumumgebung ausgesetzt angeordnet ist, wobei der Wärmesensor (56a-x) eingerichtet ist, ein Vorhandensein des Exkretionsprodukt wahrzunehmen, wenn eine Temperatur des Exkretionsprodukts größer ist als eine Raumtemperatur in der Raumumgebung, oder wobei mindestens einer der Sensoren ein Feuchtigkeitssensor (60a-x) ist, der in der Puppe (18) angeordnet ist, wobei der Feuchtigkeitssensor (60a-x) eingerichtet ist, eine Feuchtigkeit des Exkretionsprodukts wahrzunehmen.

9. Das System (10) gemäß einem der vorherigen Ansprüche, wobei der Computer (14) eingerichtet ist, das Verteilungsmuster aufzunehmen, welches durch das Exkretionsprodukt erzeugt wird, welches von den Sensoren wahrgenommen wird.

10. Das System (10) gemäß Anspruch 9, wobei der Computer (14) Mittel zum Wiedergeben des gespeicherten Verteilungsmusters für einen Benutzer beinhaltet, um das Verteilungsmuster zu analysieren, welches in Echtzeit gebildet wird.

11. Das System (10) gemäß Anspruch 1, 9 oder 10, welches des Weiteren ein Mittel zum Programmieren des Computers (14) umfasst, um das Exkretionsprodukt in das Verteilungsmuster umzuwandeln, wobei das Mittel zum Programmieren vorzugsweise ein Softwareprogramm beinhaltet, welches eingerichtet ist, das Verteilungsmuster in Echtzeit aufzunehmen und das Verteilungsmuster zur Analyse wiederzugeben.

12. Das System (10) gemäß einem der vorherigen Ansprüche, welches des Weiteren eine Insultöffnung umfasst, die eingerichtet ist, simuliertes menschliches Urin von der Puppe (18) in die Unterwäsche zu insultieren, wobei das simulierte menschliche Urin vorzugsweise ein Verdunkelungsmittel beinhaltet, um das Wahrnehmen durch die Sensoren zu beeinflussen.

13. Verfahren zum Bestimmen der Verteilung biologischer Abfallprodukte in einer Unterwäsche, wobei das Verfahren die Schritte umfasst:
Bereitstellen einer Puppe (18), die eine Entleerungsöffnung (26) dahindurch definiert, wobei die Entleerungsöffnung (26) eingerichtet ist, ein simuliertes menschliches Exkretionsprodukt aus der Puppe (18) auszuscheiden;
**gekennzeichnet durch** die Schritte:
Anordnen einer Vielzahl von Sensoren um die Entleerungsöffnung (26), wobei jeder Sensor eingerichtet ist zum Wahrnehmen des Exkretionsprodukts, wenn das Exkretionsprodukt aus der Entleerungsöffnung (26) in eine Unterwäsche ausgeschieden wird, die um die Puppe (18) angeordnet ist, wobei mindestens einer der Sensoren eine Photodiode (40a-x) ist, welche im Wesentlichen bündig mit einer simulierten Hautoberfläche (22) der Puppe (18) ist, so dass eine Emission von einer Lichtquelle **durch** das Exkretionsprodukt beeinflusst wird, wenn sich das Exkretionsprodukt zwischen die Photodiode (40a-x) und die Lichtquelle bewegt, und wobei die Photodiode (40a-x) unter einer transparenten Beschichtung angeordnet ist, wobei die transparente Beschichtung um die simulierte Hautoberfläche (22) angeordnet ist; und
Bereitstellen eines Computers (14) in Kommunikation mit den Sensoren, wobei der Computer (14) eingerichtet ist, das wahrgenommene Exkretionsprodukt in ein Verteilungsmuster umzuwandeln, welches in der Unterwäsche definiert ist.

14. Das Verfahren gemäß Anspruch 13, wobei die Sensoren eine Vielzahl an Photodioden (40a-x), eine Vielzahl an Wärmesensoren (56a-x), eine Vielzahl an Feuchtigkeitssensoren (60a-x) oder eine Kombination davon sind.

15. Das Verfahren gemäß Anspruch 13 oder 14, welches des Weiteren den Schritt des Injizierens eines Verdunklungsmittels in das Exkretionsprodukt umfasst, so dass das Exkretionsprodukt eine opake Erscheinung aufweist, um die Emission zu blocken.

16. Das Verfahren gemäß einem der Ansprüche 13-15, welches des Weiteren die Schritte des Aufnehmens eines Verteilungsmusters des Exkretionsprodukts umfasst, welches durch die Sensoren wahrgenommen wird, und des Wiedergebens des Verteilungsmusters des Exkretionsprodukts für einen Benutzer, um das Verteilungsmuster zu analysieren.

17. Das Verfahren gemäß einem der Ansprüche 13-16, welches des Weiteren den Schritt umfasst, die Puppe (18) in Bewegung zu setzen, während das Exkretionsprodukt aus der Entleerungsöffnung (26) in die Unterwäsche ausgeschieden wird.

## Revendications

1. Système (10) pour analyser des déchets biologiques dans un sous-vêtement, le système (10) comprenant :
un mannequin (18) ayant une surface de peau (22) simulée et définissant, au travers de celle-ci, un orifice d'évacuation (26), l'orifice d'évacuation (26) étant configuré pour éliminer un produit d'excrétion humain simulé depuis le mannequin (18), **caractérisé par**
une pluralité de capteurs disposés autour de l'orifice d'évacuation (26), chaque capteur étant configuré pour détecter le produit d'excrétion tandis que le produit d'excrétion est éliminé via l'orifice d'évacuation (26) dans un sous-vêtement disposé autour du mannequin (18) ; et
un ordinateur (14) en communication avec les capteurs, l'ordinateur (14) étant configuré pour convertir le produit d'excrétion détecté en un tracé de dispersion défini dans le sous-vêtement ;
l'un au moins des capteurs étant une photodiode (40a-x) disposée dans la surface de peau (22) du mannequin (18), la photodiode (40a-x) étant configurée pour détecter une émission de lumière extérieure à la surface de peau (22), la photodiode (40a-x) étant en outre configurée pour signaler à l'ordinateur (14) lorsque le produit d'excrétion affecte l'émission de lumière, la photodiode (40a-x) étant disposée sous un revêtement de polymère transparent, le revêtement de polymère transparent étant disposé sur la surface de peau (22).

2. Système (10) tel que défini dans la revendication 1, dans lequel le mannequin (18) est configuré pour simuler des mouvements humains.

3. Système (10) tel que défini dans la revendication 1 ou 2, dans lequel la photodiode (40a-x) affleure sensiblement la surface de peau (22).

4. Système (10) tel que défini dans l'une quelconque des revendications précédentes, dans lequel l'orifice d'évacuation (26) est sélectionné dans le groupe consistant en un anus simulé, un urètre simulé, et une combinaison des deux.

5. Système (10) tel que défini dans la revendication 3 ou 4, dans lequel le produit d'excrétion est d'apparence opaque pour bloquer l'émission lumineuse, ou d'apparence translucide pour modifier l'émission lumineuse.

6. Système (10) tel que défini dans l'une quelconque des revendications précédentes, dans lequel le produit d'excrétion est choisi parmi un simulant de fèces et un simulant de menstrues.

7. Système (10) tel que défini dans l'une quelconque des revendications 3-6, comprenant, en outre, une source de lumière disposée autour du mannequin (18), la source de lumière étant configurée pour produire l'émission de lumière pour activer la photodiode (40a-x).

8. Système (10) tel que défini dans l'une quelconque des revendications précédentes, dans lequel l'un au moins des capteurs est un capteur de chaleur (56a-x) disposé dans le mannequin (18) au moins partiellement exposé à un environnement ambiant, le capteur de chaleur (56a-x) étant configuré pour détecter la présence du produit d'excrétion lorsqu'une température du produit d'excrétion est supérieure à une température ambiante dans l'environnement ambiant, ou l'un au moins des capteurs est un capteur d'humidité (60a-x) disposé dans le mannequin (18), le capteur d'humidité (60a-x) étant configuré pour détecter une humidité du produit d'excrétion.

9. Système (10) tel que défini dans l'une quelconque des revendications précédentes, dans lequel l'ordinateur (14) est configuré pour enregistrer le tracé de dispersion en cours de production par le produit d'excrétion en cours de détection par les capteurs.

10. Système (10) tel que défini dans la revendication 9, dans lequel l'ordinateur (14) inclut des moyens pour la lecture du tracé de dispersion pour qu'un utilisateur analyse, en temps réel, le tracé de dispersion en cours de formation.

11. Système (10) tel que défini dans la revendication 1, 9 ou 10, comprenant, en outre, des moyens de programmation de l'ordinateur (14) pour transformer le produit d'excrétion en le tracé de dispersion, les moyens de programmation incluant, de préférence, un logiciel configuré pour enregistrer le tracé de dispersion en temps réel et pour permettre la lecture du tracé de dispersion pour analyse.

12. Système (10) tel que défini dans l'une quelconque des revendications précédentes, comprenant, en outre, un orifice de libération configuré pour libérer de l'urine humaine simulée depuis le mannequin (18) dans le sous-vêtement, l'urine humaine simulée incluant, de préférence, un agent obscurcissant pour affecter la détection par les capteurs.

13. Procédé de détermination de la dispersion de déchets biologiques dans un sous-vêtement, le procédé comprenant les étapes de :
fourniture d'un mannequin (18), définissant un orifice d'évacuation (26) dudit mannequin, l'orifice d'évacuation (26) étant configuré pour éliminer, depuis le mannequin (18), un produit d'excrétion humain simulé ;
**caractérisé par** les étapes de :
disposition d'une pluralité de capteurs autour de l'orifice d'évacuation (26), chaque capteur étant configuré pour détecter le produit d'excrétion tandis que le produit d'excrétion est éliminé via l'orifice d'évacuation (26) dans le sous-vêtement disposé autour du mannequin (18), l'un au moins des capteurs étant une photodiode (40a-x) affleurant sensiblement une surface de peau (22) simulée du mannequin (18) de telle sorte qu'une émission provenant d'une source de lumière est affectée par le produit d'excrétion tandis que le produit d'excrétion se déplace entre la photodiode (40a-x) et la source de lumière, la photodiode (40a-x) étant disposée sous un revêtement transparent, le revêtement transparent étant disposé autour de la surface de peau (22) simulée ; et
fourniture d'un ordinateur (14) en communications avec les capteurs, l'ordinateur (14) étant configuré pour convertir le produit d'excrétion détecté en un tracé de dispersion défini dans le sous-vêtement.

14. Procédé tel que défini dans la revendication 13, dans lequel les capteurs sont une pluralité de photodiodes (40a-x), une pluralité de capteurs de chaleur (56a-x, une pluralité de capteurs d'humidité (60a-x) ou une combinaison de ceux-ci.

15. Procédé tel que défini dans la revendication 13 ou 14, comprenant, en outre, l'étape d'injection d'un agent obscurcissant dans le produit d'excrétion de telle sorte que le produit d'excrétion a une apparence opaque pour bloquer l'émission.

16. Procédé tel que défini dans l'une quelconque des revendications 13-15, comprenant, en outre, les étapes d'enregistrement d'un tracé de dispersion du produit d'excrétion en cours de détection par les capteurs et de lecture du tracé de dispersion du produit d'excrétion pour qu'un utilisateur analyse le tracé de dispersion.

17. Procédé tel que défini dans l'une quelconque des revendications 13-16, comprenant, en outre, l'étape de mise en mouvement du mannequin (18) tandis que la produit d'excrétion est en cours d'élimination depuis l'orifice d'évacuation (26) dans le sous-vêtement.
